(19) Europäisches Patentamt
European Patent Office
Office européen des brevets

(11) Veröffentlichungsnummer: **0 087 690**
**B1**

# (12) EUROPÄISCHE PATENTSCHRIFT

(45) Veröffentlichungstag der Patentschrift: **31.10.84**

(51) Int. Cl.³: **C 07 C 85/11**, C 07 C 87/52, C 07 C 87/58

(21) Anmeldenummer: **83101551.6**

(22) Anmeldetag: **18.02.83**

## (54) Verfahren zur Herstellung von aromatischen Aminen.

(30) Priorität: **02.03.82 DE 3207475**

(43) Veröffentlichungstag der Anmeldung: **07.09.83 Patentblatt 83/36**

(45) Bekanntmachung des Hinweises auf die Patenterteilung: **31.10.84 Patentblatt 84/44**

(84) Benannte Vertragsstaaten: **BE DE FR GB IT NL**

(56) Entgegenhaltungen:
**US - A - 3 504 035**

**Chemical Abstract, Band 76, Nr. 5, 31. Januar 1972Columbus,Ohio, USA L.M.POLINSKI et al. "Aniline production by dual function catalysis" Seite 333,Spalte 2, Abstract Nr. 24811k & Ind. Eng. Chem., Prod. Res. Develop., Band 10, Nr. 4, 1971 Seiten 365-369 (Cat.D)**

(73) Patentinhaber: **BAYER AG, Konzernverwaltung RP Patentabteilung, D-5090 Leverkusen 1 Bayerwerk (DE)**

(72) Erfinder: **Stammann, Günter, Dr., Siesiusstrasse 78, D-5000 Köln 80 (DE)**
Erfinder: **Becker, Robert, Dr., Martin-Heidegger-Strasse 8, D-5090 Leverkusen 3 (DE)**
Erfinder: **Grolig, Johann, Dr., Heinrich-Luebke-Strasse 22, D-5090 Leverkusen 1 (DE)**
Erfinder: **Waldmann, Helmut, Dr., Henry-T.-von-Boettinger-Strasse 15, D-5090 Leverkusen 1 (DE)**

**EP 0 087 690 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein neues Verfahren zur Herstellung von aromatischen Aminen durch Umsetzung von aromatischen Nitroverbindungen mit Kohlenwasserstoffen.

Aromatische Amine werden bislang großtechnisch im allgemeinen durch Umsetzung der entsprechenden Nitroverbindungen mit Eisen, beispielsweise in saurem Medium oder durch katalytische Reduktion mit Wasserstoff hergestellt.

Die Umsetzung mit Eisen hat den Nachteil, daß als Koppelprodukt schwierig abzutrennende Eisenoxide entstehen, auf deren Verwendbarkeit, z. B. als Pigmente, dieses Verfahren angewiesen ist. Die Nachfrage nach aromatischen Aminen ist aber größer als die Nachfrage nach so hergestellten Eisenoxid-Pigmenten, weshalb dieses Verfahren den Bedarf an aromatischen Aminen nicht decken kann. Außerdem kann die Reaktionswärme nicht auf einem hohen Temperaturniveau gewonnen werden.

Der für die katalytische Reduktion von aromatischen Nitroverbindungen benötigte Wasserstoff wird im allgemeinen aus Kohle und Wasserdampf (Wassergasreaktion) oder durch Dampfreformieren von Kohlenwasserstoffen gewonnen. Bei der Umsetzung von Kohle mit Wasserdampf, die als endotherme Reaktion der Zufuhr von Energie bedarf, entsteht ein schwefelhaltiger Wasserstoff, der vor seinem Einsatz in Hydrierungen nach aufwendigen Methoden gereinigt werden muß, da sonst Katalysatorvergiftungen auftreten. Auch bei der Dampfreformierung von Kohlenwasserstoffen, z. B. von Erdgas bzw. Methan, handelt es sich um eine endotherme Reaktion, bei der die Energiezufuhr beispielsweise durch Unterfeuerung mit Erdgas erfolgt, wobei pro Mol zu Kohlenmonoxid und/oder Kohlendioxid und Wasserstoff umgesetztem Methan ca. 0,25 bis 0,35 Mol Methan verfeuert werden müssen. Weitere Energie ist für die Abtrennung von z. B. Kohlenmonoxid und nicht umgesetztem Methan und für die Reinigung des Wasserstoffs aufzuwenden.

Methan steht an sich kostengünstig zur Verfügung, z. B. als Hauptbestandteil des Erdgases. Ferner wird erwogen, auf chemischem Wege hergestelltes Methan, sog. SNG (= substitute natural gas), als massearmen Energieträger über Pipelines zu verteilen.

Die Herstellung von aromatischen Aminen aus den entsprechenden Nitroverbindungen könnte wesentlich einfacher und kostengünstiger als bisher durchgeführt werden, wenn es gelänge, direkt Kohlenwasserstoffe, insbesondere Methan, als Reduktionsmittel einzusetzen. Dabei könnten die umständlichen, energieaufwendigen und teuren Zwischenstufen der Herstellung und Isolierung von Wasserstoff, z. B. durch Dampfreformierung, eingespart werden.

Diesbezügliche Versuche wurden auch schon unternommen. So wird in der US-PS 2 377 071 ein solches Verfahren zur Herstellung von Aminen unter Verwendung von Chromiten unedler Metalle als Katalysatoren, beispielsweise von Eisen-, Kobalt- und Nickelchromit-Katalysatoren, beschrieben. Für die gleiche Umsetzung, allerdings in Gegenwart von Wasserdampf, werden in der US-PS 3 504 035 Katalysatoren beschrieben, welche aus einer Hydrierkomponente und einer Reformierkomponente bestehen. Die Hydrierkomponente besteht dabei im allgemeinen aus Kupfer, das zumindest während der Reaktion überwiegend in metallischer Form vorliegt, die Reformierkomponente kann ein Edelmetall oder eine Verbindung von Nickel, Eisen oder Chrom sein. Der Einsatz von Kupferchromiten oder Kupferchromit enthaltenden Katalysatoren ist hier nicht beschrieben.

Theoretische Ausführungen und weitere experimentelle Details über das Verfahren der US-PS 3 504 035 werden von Erfindern dieser US-PS in einem Zeitschrifenartikel angegeben (siehe L. M. Polinski und E. A. Harvey, Ind. Eng. Chem. Prod. Res., Develop., Vol. 10, Nr. 4 [1971], S. 365 bis 369). In diesem Zeitschriftenartikel wird auch ausgeführt, daß in der US-PS 3 504 035 beschriebene Katalysatoren nach zwei bis drei Stunden ihre Aktivität wegen Kohlenstoffablagerungen in Mengen von bis zu ca. 13% verlieren und deshalb nach dieser kurzen Zeit regeneriert werden müssen.

Nachteile bei den beiden genannten Verfahren sind die geringen Katalysatorstandzeiten von nur einigen Stunden, verursacht durch erhebliche Koksablagerungen auf dem Katalysator, insbesondere wenn zur Erzielung höherer Raum-Zeit-Ausbeuten Temperaturen um 350° C angewendet werden. Die häufige Regenerierung des Katalysators führt zu erheblichen Ausfallzeiten in der Aminproduktion. Außerdem werden die gewünschten Amine in Selektivitäten von maximal ca. 90% (bezogen auf umgesetzte Nitroverbindung) erhalten. Diese Selektivität ist noch unbefriedigend, da bei Produkten, die in Mengen wie die aromatischen Amine hergestellt werden, die Selektivität möglichst nahe bei 100% liegen sollte.

Insgesamt ist zu den bisher bekannt gewordenen Verfahren zur Herstellung von Aminen aus Nitroverbindungen unter Verwendung von Kohlenwasserstoffen als Reduktionsmittel also festzustellen, daß diese für eine technische Anwendung nicht geeignet sind.

Es wurde nun ein Verfahren zur Herstellung von primären aromatischen Aminen durch Umsetzung von aromatischen Nitroverbindungen mit Kohlenwasserstoffen gefunden, das dadurch gekennzeichnet ist, daß man Kohlenwasserstoffe mit einem Molekulargewicht bis zu 170 und einer Zusammensetzung $C_mH_n$ mit Werten von $\frac{m}{n}$ von 0,25 bis 0,60 oder Kohlenwasserstoffgemische mit einem entsprechenden mittleren Molekulargewicht und einer entsprechenden mittleren Zusammensetzung in Gegenwart von Wasser oder Wasserdampf einsetzt und die Umsetzung in Gegenwart eines Katalysators durchführt, welcher Kupfer, Chrom und daran chemisch gebundenen Sauerstoff ganz oder teilweise in Form von Kupferchromiten enthält.

Gegebenenfalls kann das erfindungsgemäße Verfahren in Gegenwart inerter Verdünnungsmittel durchgeführt werden.

Zum Einsatz in das erfindungsgemäße Verfahren geeignete aromatische Nitroverbindungen können eine oder mehrere Nitrogruppen pro Molekül enthalten, welche direkt an ein carbocyclisches oder heterocyclisches aromatisches Ringsystem gebunden sind. Das Ringsystem kann z. B. aus 5 bis 20 Ringgliedern bestehen und außer Kohlenstoffatomen z. B. bis zu 4 Heteroatome im Ringsystem enthalten. Derartige Heteroatome können z. B. Stickstoff, Sauerstoff oder Schwefel sein. Enthält die aromatische Nitroverbindung mehr als einen Ring, so können diese als kondensierte Ringe vorliegen oder auf verschiedene Weise miteinander verknüpft sein, beispielsweise durch eine Kohlenstoff-Kohlenstoff- oder Kohlenstoff-Stickstoff-Bindung. Es können auch zwei oder mehr Ringsysteme durch Heteroatome, z. B. durch Stickstoff-, Sauerstoff- oder Schwefelatome oder durch eine Oxogruppe (—CO—) miteinander verbunden sein.

Die aromatischen Nitroverbindungen können außer Nitrogruppen gegebenenfalls einen oder mehrere weitere Substituenten aufweisen. Als solche Substituenten kommen z. B. Hydroxygruppen und primäre Aminogruppen, sowie, falls das erfindungsgemäße Verfahren bei relativ niedrigen Temperaturen durchgeführt wird, Halogen-, Cyano-, Carbonsäure- und Sulfonsäuregruppen in Frage. Auch durch Kohlenwasserstoffreste, z. B. Alkyl- oder Cycloalkylreste substituierte aromatische Nitroverbindungen können eingesetzt werden, beispielsweise Nitrotoluol, wobei ebenfalls in hohen Selektivitäten primäre aromatische Amine gebildet werden. Hierbei können aber auch Dealkylierungen stattfinden.

Die Zahl der Nitrogruppen pro Molekül aromatischer Nitroverbindung kann z. B. 1 bis 3 betragen, wobei pro Einzelring oder pro Teilring eines Ringsystems vorzugsweise nicht mehr als 2 Nitrogruppen vorhanden sind.

Für das erfindungsgemäße Verfahren geeignete aromatische Nitroverbindungen sind z. B. solche des Benzols, Naphthalins, Diphenyls, Anthrachinons, Phenanthrens, Furans, Thiophens, Pyridins, Benztriazols, Diphenylethers, Carbazols und Benzothiazols. Vorzugsweise werden Nitroverbindungen des Benzols, Naphthalins, Phenols, Anilins und 5- oder 6gliedriger aromatischer Heterocyclen eingesetzt.

Beispiele für geeignete aromatische Nitroverbindungen sind: Nitrobenzol, Nitronaphthaline, 4-Nitrodiphenyl, Nitroanthrachinon, Nitrophenanthrene, 2-Nitrofuran, 2-Nitrothiophen, 3-Nitropyridin, 5-Nitro-1H-benzotriazol, 2-Nitrodiphenylether, die isomeren Dinitrobenzole, die isomeren Nitroaniline, p-Nitrobenzoesäure, m-Nitrobenzoesäure, die isomeren Nitrophenole, p-Nitrochlorbenzol, 5-Nitro-2-chlorbenzonitril, 3,4-Dichlornitrobenzol, 2-Nitro-1,4-phenylendiamin, 2,4-Dinitroanilin, 2,4-Dinitrochlorbenzol, 1,8-Dinitro-4,5-dioxianthrachinon, 3-Nitrophthalsäureimid, 3,6-Dinitro-9H-carbazol und 2-[(2,4-Dinitrophenyl)-thio]-benzothiazol.

Bevorzugte Nitroverbindungen zum Einsatz in das erfindungsgemäße Verfahren sind Nitrobenzol, die isomeren Dinitrobenzole, 1-Nitronaphthalin, 2-Nitronaphthalin, die isomeren Nitrophenole, die isomeren Nitroaniline und Mononitroderivate von 5- und 6gliedrigen aromatischen Heterocyclen.

Als aromatische Nitroverbindungen werden besonders bevorzugt Nitrobenzol und 1,3-Dinitrobenzol in das erfindungsgemäße Verfahren eingesetzt.

Bei der Durchführung des erfindungsgemäßen Verfahrens können als Nebenprodukte in geringen Mengen Azoverbindungen, Azoxiverbindungen und Hydrazoverbindungen auftreten, eventuell auch Nitrosoverbindungen. Diese Verbindungen enthalten je Stickstoffatom einen aromatischen Rest, welcher der eingesetzten aromatischen Nitroverbindung entspricht. Die genannten, als Nebenprodukte gegebenenfalls auftretenden Stickstoffverbindungen, verursachen jedoch keine besonderen Probleme, denn sie können in das erfindungsgemäße Verfahren zurückgeführt werden und führen, gegebenenfalls nach mehrmaligem Durchsatz auch zu den gewünschten Verfahrensprodukten, wenn sie in geringen Mengen zusammen mit den eingesetzten aromatischen Nitroverbindungen erfindungsgemäß umgesetzt werden.

Die zuvor beschriebenen Nebenprodukte können z. B. bis maximal 10 Gew.-%, vorzugsweise im Bereich von 1 bis 5 Gew.-%, der frisch einzusetzenden aromatischen Nitroverbindung zugesetzt werden, beispielsweise nachdem man diese Nebenprodukte aus der Sumpfphase bei der Destillation der Aminverbindungen abgetrennt hat. Derartige Nebenprodukte stellen in den genannten Mengen gemeinsam mit den entsprechenden Nitroverbindungen ein für das erfindungsgemäße Verfahren geeignetes Einsatzmaterial dar.

Die erfindungsgemäß als zweites Ausgangsprodukt einzusetzenden Kohlenwasserstoffe sind solche mit einem Molekulargewicht bis zu 170 und mit einer Zusammensetzung $C_mH_n$ mit Werten von $\frac{m}{n}$ von 0,25 bis 0,60 oder Kohlenwasserstoffgemische mit einem mittleren Molekulargewicht $\overline{M}$ bis zu 170 und mit einer mittleren Zusammensetzung $C_mH_n$ mit Werten von $\frac{m}{n}$ von 0,25 bis 0,60. Diese Beschränkungen gelten bei Kohlenwasserstoffgemischen nur für das gesamte Gemisch, einzelne Komponenten des Gemisches können auch ein höheres Molekulargewicht als 170 und einen höheren $\frac{m}{n}$-Wert als 0,60 haben.

Für einzelne Kohlenwasserstoffe bedeutet m die Zahl der C-Atome und n die Zahl der H-Atome in der Summenformel der Kohlenwasserstoffverbindung. Für Kohlenwasserstoffgemische bezeichnen m und

n analog die entsprechende mittlere Anzahl der C- bzw. H-Atome in einer gemittelten Summenformel $C_mH_n$. Das mittlere Molekulargewicht $\overline{M}$ und die mittlere Zusammensetzung $C_mH_n$ eines Kohlenwasserstoffgemisches sind über die Beziehung

$$\overline{M} = \sum_{i=1}^{i} X_i \, (12\, m_i + n_i) = 12\, m + n$$

verknüpft, wobei i die einzelnen Kohlenwasserstoffe in einem Kohlenwasserstoffgemisch kennzeichnet und $X_i$ den Molenbruch der Komponente i im Kohlenwasserstoffgemisch darstellt. Bei komplexen Gemischen kann $\overline{M}$ auch nach üblichen analytischen Methoden bestimmt werden. Unter Einbeziehung der C,H-Elementaranalyse lassen sich dann $\frac{m}{n}$ bzw. $C_mH_n$ für das Gemisch errechnen.

Die Struktur der Kohlenwasserstoffe ist ohne besondere Bedeutung, es können, innerhalb der oben angegebenen Begrenzungen, z. B. linear-kettenförmige, verzweigt-kettenförmige und/oder cyclische Kohlenwasserstoffe, sowie gesättigte und ungesättigte Kohlenwasserstoffe eingesetzt werden. Vorzugsweise werden gesättigte Kohlenwasserstoffe und/oder solche ungesättigten Kohlenwasserstoffe eingesetzt, die Methylgruppen und/oder innenständige Methylengruppen enthalten. Sind in Kohlenwasserstoffgemischen unsubstituierte aromatische Kohlenwasserstoffe enthalten, so verhalten sich diese weitgehend inert. Alkylsubstituierte aromatische Kohlenwasserstoffe werden hingegen unter den Bedingungen des erfindungsgemäßen Verfahrens ganz oder teilweise dealkyliert.

In den Kohlenwasserstoffen können geringe Mengen von Verbindungen vorhanden sein, die z. B. Stickstoff, Sauerstoff oder Schwefel in gebundener Form enthalten. Derartige Verbindungen stören das erfindungsgemäße Verfahren nicht, wenn sich aus der Elementaranalyse der Kohlenwasserstoffe ergibt, daß die Anteile von Stickstoff und Schwefel jeweils 0,1 Gew.-% nicht übersteigen. Chemisch gebundener Sauerstoff ist auch in höheren Gewichtsanteilen tolerierbar, z. B. bis zu 2 Gew.-%. Werden gasförmige Kohlenwasserstoffe eingesetzt, so können diese, insbesondere wenn natürliches Erdgas verwendet wird, inerte Gase enthalten, beispielsweise Stickstoff, Kohlendioxid und/oder Edelgase. Steht als Kohlenwasserstoff auf chemischem Weg hergestelltes Methan (SNG) zur Verfügung, so kann dieses aufgrund einer unvollständigen Methanisierungsreaktion noch einige Volumenprozente an Kohlenmonoxid und/oder Wasserstoff enthalten. Solche Gasgemische können ohne Vorreinigung direkt in das erfindungsgemäße Verfahren eingesetzt werden.

Geeignete Kohlenwasserstoffe sind innerhalb der angegegebenen Beschränkung beispielsweise solche mit bis zu 12 C-Atomen, vorzugsweise mit bis zu 8 C-Atomen, auch in Form von Gemischen. Geeignete Kohlenwasserstoffgemische sind z. B. sog. Leichtbenzin, Flüssiggas (LPG) und/oder Naturgasbenzin (s. F. Asinger, Die Petrolchemische Industrie, Akademie-Verlag Berlin [1971], Band 1, Seiten 4—11). Von den ungesättigten Kohlenwasserstoffen sind solche bevorzugt, die bis zu 12 C-Atome, vorzugsweise bis zu 8 C-Atome aufweisen und pro Doppelbindung mindestens eine Methylgruppe und/oder innenständige Methylengruppe enthalten. Beispiele für Einzelverbindungen, die jeweils für sich allein oder in beliebigen Mischungen untereinander eingesetzt werden können sind Methan, Ethan, Propan, Butan, Pentan, Hexan, Heptan, Octan, Nonan, Decan, Undecan und Dodecan, soweit dies möglich ist in beliebigen Verzweigungsgraden, entsprechende gesättigte cyclische, auch alkylsubstituierte Kohlenwasserstoffe und entsprechende acyclische und cyclische Monoolefine mit innenständigen bzw. ringständigen Methylengruppen.

Bevorzugte Kohlenwasserstoffe für das erfindungsgemäße Verfahren sind Kohlenwasserstoffe oder Kohlenwasserstoffgemische mit einem Siedepunkt bzw. Siedebereich bis zu 130° C (bei Normaldruck) und einer (mittleren) Zusammensetzung $C_mH_n$, wobei $\frac{m}{n}$ im Bereich von 0,25 bis 0,45 liegt.

Besonders bevorzugt werden Methan, natürliches Erdgas, welches ein sog. trockenes oder ein sog. nasses Erdgas sein kann (s. F. Asinger a.a.O.) und als Inertbestandteile Stickstoff, Kohlendioxid und/oder Edelgase enthalten kann und/oder SNG (substitute natural gas) als Kohlenwasserstoffe eingesetzt.

Das erfindungsgemäße Verfahren wird in Gegenwart eines Katalysators durchgeführt, der mindestens die Komponenten Kupfer, Chrom und chemisch gebundenen Sauerstoff enthält, wobei diese Komponenten ganz oder teilweise in Form von Kupferchromiten vorliegen. Das Kupfer sollte überwiegend in den Oxidationsstufen +1 und/oder +2 vorliegen. Geringe Anteile davon können auch in metallischer Form vorliegen. Das Chrom sollte in kationischer Form vorliegen, vorzugsweise in der Oxidationsstufe +3. Die kationischen Katalysatorkomponenten können z. B. als physikalische Gemische der entsprechenden Metalloxide und/oder als Mischoxide eingesetzt werden, wobei aber Kupfer, Chrom und Sauerstoff zumindest teilweise in Form von Kupferchromiten vorliegen müssen. Beispielsweise liegen mindestens 15 Gew.-%, vorzugsweise mehr als 30 Gew.-%, des Katalysators als Kupferchromit vor.

Bevorzugte Katalysatoren für das erfindungsgemäße Verfahren sind Mischoxide aus Kupferoxiden und Chrom(III)-oxid, z. B. die Kupferchromite der Formeln $CuCr_2O_4$ und/oder $Cu_2Cr_2O_4$, sowie Gemische dieser Verbindungen mit Kupferoxiden und/oder Chromoxiden und/oder metallischem Kupfer. Die Gesamtzusammensetzung bevorzugter Katalysatoren kann z. B. durch die summarische Formel

$$xCuO \cdot (1-x)Cr_2O_3$$

beschrieben werden, wobei x Gewichtsanteile bedeutet und Werte im Bereich von 0,2 bis 0,9 annehmen kann. Besonders bevorzugt sind Katalysatoren dieser Formel, bei denen x für Werte im Bereich von 0,4 bis 0,8 steht. Liegt Kupfer nicht oder nicht nur in der Oxidationsstufe +2 vor, wie es die vorstehende Formel vorsieht, so wird unabhängig von der Oxidationsstufe das Verhältnis Kupfer zu Chrom vorzugsweise entsprechend der vorgenannten Zusammensetzung gewählt.

Der erfindungsgemäß zu verwendende Katalysator kann gegebenenfalls eine oder mehrere weitere Komponenten enthalten. Diese Komponenten können z. B. aus den Elementen des Periodensystems mit den Ordnungszahlen 25 bis 28, 30 und 56 (im folgenden werden diese auch mit dem Symbol $M_I$ bezeichnet) und/oder aus den Elementen des Periodensystems mit den Ordnungszahlen 44 bis 47 und 75 bis 79 (im folgenden werden diese auch mit dem Symbol $M_{II}$ bezeichnet) ausgewählt werden.

Die weiteren Katalysatorkomponenten des Typs $M_I$ liegen vorzugsweise in oxidischer Form vor. Das bedeutet, der erfindungsgemäß zu verwendende Katalysator kann auch als Mischoxid und/oder Gemisch von Oxiden der Metalle Kupfer, Chrom und $M_I$ vorliegen, wobei $M_I$ vorzugsweise in der Oxidationsstufe +2 vorliegt und Kupfer, Chrom und Sauerstoff zumindest teilweise in Form von Kupferchromiten vorliegen. Die meisten der Metalle $M_I$ können in Verbindungen der Formel $CuCr_2O_4$ mit Spinellstruktur, welche erfindungsgemäß einsetzbare Katalysatoren sind, das Kupfer unter Erhaltung des Strukturtyps teilweise ersetzen. Solche Mischoxide stellen ebenfalls erfindungsgemäß zu verwendende Katalysatoren dar. Ihre Struktur kann beispielsweise durch Röntgenaufnahmen nach Debye-Scherrer charakterisiert werden, bei welchen sich, trotz des Vorhandenseins der Komponente $M_I$ allenfalls nur geringe Unterschiede zu denen von $CuCr_2O_4$ feststellen lassen. Eine bevorzugte weitere Katalysatorkomponente des Typs $M_I$ ist Barium.

Die Mitverwendung einer Komponente $M_I$ im erfindungsgemäß einzusetzenden Katalysator kann zur Erzielung besserer mechanischer und thermischer Stabilität, aber auch alleine aus Kostengründen vorteilhaft sein. Die Gesamtzusammensetzung des Katalysators kann bei Mitverwendung einer Komponente $M_I$ z. B. durch die summarische Formel

$$xCuO \cdot yM_IO \cdot (1-x-y)Cr_2O_3$$

angegeben werden, wobei x und y Gewichtsanteile bedeuten, die Summe von x plus y 0,2 bis 0,9 beträgt und y kleiner, höchstens gleich x ist. Vorzugsweise beträgt die Summe von x plus y 0,4 bis 0,8 und vorzugsweise ist y kleiner als die Hälfte von x.

Die wahlweise zu verwendende Katalysatorkomponente $M_{II}$ liegt im Katalysator vorzugsweise in metallischer Form vor. Ihre Mitverwendung erlaubt im Vergleich zu Katalysatoren ohne Komponente $M_{II}$ im allgemeinen höhere Raum-Zeit-Ausbeuten des Verfahrensproduktes, gegebenenfalls bei niedrigeren Temperaturen und/oder längere Katalysatorstandzeiten zwischen den Regenerationsphasen. Die Menge der Katalysatorkomponente $M_{II}$ kann z. B. bis zu 5 Gew.-%, vorzugsweise bis zu 1 Gew.-%, betragen, jeweils bezogen auf den fertigen Katalysatorkörper. Bevorzugte weitere Katalysatorkomponenten des Typs $M_{II}$ sind Palladium und Silber.

Katalysatoren mit den Komponenten Kupfer, Chrom und daran chemisch gebundenen Sauerstoff, die ganz oder teilweise in Form von Kupferchromiten vorliegen, und gegebenenfalls Komponenten des Typs $M_I$ enthalten, können gemäß den üblichen Methoden zur Herstellung von Mischoxiden erhalten werden. Solche Methoden sind schon zusammenfassend beschrieben worden (siehe Ph. Courty und C. Marcilly, Revue de l'Institut Français du Petrole, Vol. 33, Nr. 1 (1978), S. 83 bis 100 und B. Delmon, P. A. Jacobs and G. Poncelet (Editors) in »Preparation of Catalysts«, Amsterdam, Oxford, New York – Elsevier [1976]). Für das erfindungsgemäße Verfahren geeignete Kupferchromittypen sind auch kommerziell erhältlich und werden beispielsweise von Alfa Products (in Deutschland vertrieben von Ventron, Karlsruhe), Katalog 1981, Nr. 89 932, 11 842, 11 843, 11 844 und 11 845; von Bayer AG (Katalog Nr. 9127) und von Strem Chemicals (Newburyport, Massachusetts, USA), Katalog 1980–81, Nr. 290 370 angeboten.

Zur Herstellung eines Katalysators, welcher Komponenten des Typs $M_{II}$ enthält, können Salze der Elemente $M_{II}$, beispielsweise Chloride, Nitrate, Acetylacetonate, Carbonate oder deren Salze von organischen Säuren in der entsprechenden Menge bei den Sinter- oder Fällungsverfahren zur Herstellung der Mischoxide zugesetzt werden. Ebenso können Hydroxide oder Oxide der Metalle $M_{II}$ verwendet werden. Danach kann, um metallisches $M_{II}$ zu erhalten, eine reduktive Behandlung durchgeführt werden.

Eine andere Methode zum Aufbringen von Komponenten des Typs $M_{II}$ besteht im Tränken des Oxidpulvers oder der fertig geformten Katalysatorkörper mit einer Lösung von Salzen von $M_{II}$, wobei das Lösungsmittel dann ganz oder teilweise verdampft und anschließend das Metall $M_{II}$ reduktiv abgeschieden werden kann.

Die reduktive Abscheidung von $M_{II}$ erfolgt vorzugsweise bei etwa 100 bis 400° C mit reduzierenden Gasen, beispielsweise mit Wasserstoff, Kohlenmonoxid oder gasförmigen Kohlenwasserstoffen oder Gemischen dieser Gase. Die gewünschte Abscheidung von $M_{II}$ und Aktivierung des Katalysators kann auch unter den Bedingungen des erfindungsgemäßen Verfahrens erreicht werden. Eine direkte Vermischung der Metalle $M_{II}$ in Pulverform mit den übrigen Katalysatorbestandteilen ist auch möglich, aber

im allgemeinen weniger vorteilhaft.

Für das erfindungsgemäße Verfahren wird der Katalysator, unabhängig davon, wie er innerhalb des vorbeschriebenen Rahmens zusammengesetzt ist, im allgemeinen als Pulver oder in Gestalt von Formkörpern, die z. B. durch Pressen, Granulieren oder Extrudieren entstehen, verwendet.

In einer bevorzugten Ausführungsform der Erfindung wird der Katalysator in Pellets oder Kugeln eingesetzt. Um eine ausreichende mechanische und thermische Stabilität der Formkörper zu erreichen ist es vorteilhaft, den wirksamen Katalysator mit üblichen inerten Trägern und/oder Zusatzstoffen, z. B. Aluminiumoxid, Siliciumdioxid, Silikaten, Tonerden, Graphit oder organischen Bindemitteln, z. B. Stearate und/oder Leim zu verarbeiten. Diese und ähnliche Hilfsstoffe können auch in den genannten kommerziell erhältlichen Katalysatortypen enthalten sein.

Um poröse Katalysatortypen zu erhalten, können übliche Porosierungsmittel, z. B. Carbonate, Oxalate oder flüchtige Ammoniumsalze, bei der Katalysatorherstellung mit verwendet werden, die sich bei einer thermischen Behandlung des Katalysators verflüchtigen. Für den gleichen Zweck können wasserlösliche Alkali- oder Erdalkaliverbindungen eingesetzt werden, die durch Behandlung mit Wasser oder im erfindungsgemäßen Verfahren selbst aus dem Katalysatorkörper ausgewaschen werden können.

Geeignete Katalysatorkörper können auch durch Aufbringen aller Katalysatorkomponenten auf vorgeformte inerte Trägermaterialien hergestellt werden. Ein solches Verfahren zur Herstellung von Kupferchromit auf einem inerten Trägermaterial wird beispielsweise von G. Laidig, D. Hönicke und K. Griesbaum, Erdöl & Kohle, Erdgas, Petrochemie, 34 (1981), Nr. 8, S. 329 bis 336, Abschnitt 3.1 beschrieben.

Aus Kostengründen ist es vorteilhaft, den Anteil der aktiven Katalysatorkomponenten am fertigen Katalysatorkörper möglichst gering zu halten. Es können deshalb z. B. bis zu 70 Gew.-%, vorzugsweise bis zu 30 Gew.-% der genannten inerten Zusatz- und Hilfsstoffe eingesetzt werden (bezogen auf den gesamten Katalysatorkörper). Bei Katalysatorkörpern, welche im Tränkverfahren oder als Schalenkontakte hergestellt werden, kann der Gewichtsanteil des Trägers auch höher sein, z. B. bis zu 90 Gew.-% (bezogen auf den gesamten Katalysatorkörper). Es kann auch vorteilhaft sein, die Katalysatorschüttung mit Inertkörpern zu verdünnen, was im allgemeinen auch die Temperaturführung erleichtert.

Das erfindungsgemäße Verfahren wird in Gegenwart von Wasser, vorzugsweise in Gegenwart von Wasserdampf durchgeführt. Es kann ausreichend sein, nur in Gegenwart des sich während der Reaktion bildenden Wassers zu arbeiten. Vorzugsweise wird Wasser oder Wasserdampf zugeführt, beispielsweise das 0,1- bis 3fache des Gewichts der eingesetzten aromatischen Nitroverbindung. Besonders bevorzugt arbeitet man in Gegenwart der 0,5- bis 1,5fachen Menge Wasser oder Wasserdampf, bezogen auf das Gewicht der eingesetzten aromatischen Nitroverbindung.

Das erfindungsgemäße Verfahren kann allein mit den Ausgangsstoffen aromatischer Nitroverbindungen und Kohlenwasserstoff bzw. Kohlenwasserstoffgemischen und in Gegenwart von Wasser oder Wasserdampf und dem Katalysator durchgeführt werden. Zur Verlängerung der Katalysatorstandzeit, zur Selektivitätserhöhung und zur besseren Einstellung der gewünschten Verweilzeit kann die Mitverwendung eines oder mehrerer inerter Verdünnungsmittel, insbesondere bei kontinuierlicher Arbeitsweise, vorteilhaft sein. Als inerte Verdünnungsmittel kommen z. B. unsubstituierte aromatische Kohlenwasserstoffe, Stickstoff, Edelgase und Kohlendioxid in Frage. Als inert werden in diesem Zusammenhang solche Stoffe bezeichnet, die gemäß der weiter unten angeführten Bruttogleichung des erfindungsgemäßen Verfahrens nicht oder nur in sehr untergeordnetem Maße verbraucht werden. Ein bevorzugtes inertes Verdünnungsmittel ist Stickstoff.

Das erfindungsgemäße Verfahren kann in der Gasphase und/oder in der Flüssigphase durchgeführt werden. Vorzugsweise wird es in der Gasphase durchgeführt.

Das erfindungsgemäße Verfahren wird bei erhöhter Temperatur durchgeführt. Es kann bei Unter-, Normal- oder Überdruck, kontinuierlich oder diskontinuierlich durchgeführt werden. Für den Fall der Totaloxidation des eingesetzten Kohlenwasserstoffs erfolgt die Umsetzung gemäß nachstehender, angenähert geltender allgemeiner Gleichung:

$$R-NO_2 + u \cdot C_mH_n \longrightarrow R-NH_2 + u \cdot mCO_2 + \frac{u(n-2m)}{3} H_2O$$

In dieser Gleichung bedeutet R einen aromatischen Rest und u steht für $\frac{6}{4m+n}$. Für m und n gelten die weiter oben angegebenen Beziehungen.

Die Reaktionstemperatur bei dem erfindungsgemäßen Verfahren liegt beispielsweise im Bereich von 250 bis 450° C. Je nach Art der Einsatzstoffe und abhängig vom Druck kann die erfindungsgemäße Umsetzung in flüssiger Phase oder in der Gasphase durchgeführt werden. Vorzugweise werden Drucke im Bereich Normaldruck bis zu 50 bar, besonders bevorzugt bis zu 10 bar, angewendet und die Temperatur so gewählt, daß zumindest der größte Teil der aromatischen Nitroverbindung und der Kohlenwasserstoffe während der Reaktion gasförmig vorliegt.

Das erfindungsgemäße Verfahren wird im allgemeinen unter Einsatz eines Überschusses an Kohlenwasserstoffen durchgeführt. Vorzugsweise werden die Kohlenwasserstoffe in einer Menge eingesetzt, die der 1,2- bis 20fachen Menge entspricht, die stöchiometrisch gemäß der oben angegebenen Gleichung erforderlich ist. Inerte Verdünnungsmittel können in Mengen bis zu 90 Gew.-%, vorzugsweise bis zu 30 Gew.-% eingesetzt werden (bezogen auf das gesamte Einsatzgemisch).

Wenn man das erfindungsgemäße Verfahren in flüssiger Phase durchführt, kann der Katalysator beispielsweise als Suspension oder im Rieselbett eingesetzt werden. Für die bevorzugte Ausführungsform in der Gasphase ist z. B. die Arbeitsweise im Fließbett, Wirbelbett oder Festbett geeignet. Besonders bevorzugt ist das Festbettverfahren unter Verwendung von Rohrreaktoren.

Die Reaktionszeit für das erfindungsgemäße Verfahren hängt stark von den sonstigen Reaktionsbedingungen ab. Beim Arbeiten in flüssiger Phase kann sie beispielsweise einige Minuten bis wenige Stunden betragen. Bei der bevorzugten Arbeitsweise in der Gasphase kann sie beispielsweise $^1/_{10}$ Sekunde bis 1 Minute betragen. Bei kontinuierlicher Arbeitsweise kann eine entsprechende mittlere Verweilzeit durch den Überschuß an Kohlenwasserstoffen, durch die Menge an Wasser bzw. Wasserdampf und/oder die Menge an inerten Verdünnungsmitteln eingestellt werden.

Wenn der Katalysator im Laufe der Zeit in der Aktivität und/oder Selektivität abfällt, kann er regeneriert werden. Hierzu kann man molekularen Sauerstoff enthaltende Gase, vorzugsweise Luft, bei erhöhter Temperatur über den Katalysator leiten. Es ist vorteilhaft, die Regeneration in Gegenwart von Wasserdampf durchzuführen.

Beispielsweise kann der Katalysator direkt in einem Reaktionsrohr unter Beibehaltung der Reaktionstemperatur nach Zwischenspülung mit Stickstoff in einigen Stunden regeneriert werden. Eine reduktive Nachbehandlung, vorzugsweise mit einem gasförmigen Kohlenwasserstoff, insbesondere Methan, ist vorteilhaft. Diese reduktive Nachbehandlung kann auch dadurch erreicht werden, daß bei vollem Einsatz des erfindungsgemäß einzusetzenden Kohlenwasserstoffs bzw. Kohlenwasserstoffgemisches der Einsatz der erfindungsgemäß einzusetzenden aromatischen Nitroverbindung nur langsam auf den Sollwert gebracht wird. Der Katalysator kann nach einigen abwechselnden Reaktions- und Regenerationszyklen an Aktivität zunehmen.

Nach der Durchführung des erfindungsgemäßen Verfahrens und gegebenenfalls nach Abtrennung des Katalysators fällt das Reaktionsgemisch, in Abhängigkeit von den gewählten Reaktionsbedingungen, entweder vollständig gasförmig oder als Gemisch einer gasförmigen und einer oder mehreren flüssigen Phasen an. Zur Aufarbeitung der Reaktionsgemische kühlt man diese, unabhängig davon wie sie anfallen, im allgemeinen ab, und zwar im allgemeinen mindestens soweit, daß auch das im Reaktionsgemisch vorhandene Wasser kondensiert. Geeignete Temperaturen, auf die das Reaktionsgemisch abgekühlt werden kann, sind beispielsweise solche im Bereich von 10 bis 90° C, bevorzugt solche im Bereich von 30 bis 60° C. Nach einer solchen Abkühlung liegt dann im allgemeinen eine, im wesentlichen Kohlendioxid und gegebenenfalls gasförmige Inerte enthaltende Gasphase und mindestens eine wäßrige und eine organische flüssige Phase vor, die dann voneinander getrennt werden.

Die weitere Aufarbeitung kann dann beispielsweise wie folgt durchgeführt werden: Aus der organischen Phase wird das gebildete Amin durch eine übliche Trennmethode isoliert, vorzugsweise durch Destillation. Nicht umgesetzte aromatische Nitroverbindungen und die gegebenenfalls als Nebenprodukte entstandenen Nitroso-, Azo-, Azoxi- und Hydrazoverbindungen können nach ihrer Abtrennung (sie befinden sich im allgemeinen im Sumpf der Destillationskolonne, in der das gebildete Amin über Kopf abgetrennt wird) mit frischer aromatischer Nitroverbindung versetzt und wieder in das erfindungsgemäße Verfahren eingesetzt und so zu den gewünschten Endprodukten umgesetzt werden. Wurden aromatische Nitroverbindungen mit mehr als einer Nitrogruppe pro Molekül eingesetzt, so können als Nebenprodukte auch Verbindungen entstanden sein, welche Amino- und Nitrogruppen enthalten. Diese Verbindungen fallen im allgemeinen zusammen mit den zuvor beschriebenen Nebenprodukten an und können zusammen mit diesen rückgeführt und zu den gewünschten Endprodukten umgesetzt werden.

In der organischen Phase können, wenn andere als die besonders bevorzugten Kohlenwasserstoffe bzw. Kohlenwasserstoffgemische eingesetzt wurden, Kohlenwasserstoffe mit erhöhtem Olefin- und/oder Aromatengehalt vorhanden sein. Diese Kohlenwasserstoffe können von den Verfahrensprodukten und nicht umgesetzter aromatischer Nitroverbindung z. B. durch Destillation abgetrennt und ebenfalls zurückgeführt werden. Es ist vorteilhaft, vor der Rückführung gegebenenfalls vorhandene aromatische Kohlenwasserstoffe abzutrennen, beispielsweise durch eine Extraktion. Das, gegebenenfalls von Aromaten befreite Kohlenwasserstoffgemisch kann dann mit frischen Kohlenwasserstoffen versetzt und wieder in das erfindungsgemäße Verfahren eingesetzt werden. Dabei ist zu beachten, daß die weiter oben genannte Gesamtzusammensetzung für die einzusetzenden Kohlenwasserstoffe eingehalten wird.

Die wäßrige Phase kann einen geringen Anteil des als Verfahrensprodukts gewünschten aromatischen Amins enthalten. Dieser kann entweder destillativ oder extraktiv entfernt werden. Besonders günstig ist es, die Wasserphase mit den einzusetzenden aromatischen Nitroverbindungen zu extrahieren. Das extrahierte Amin kann dann entweder von der Nitroverbindung durch Destillation getrennt oder zusammen mit dieser nochmals das erfindungsgemäße Verfahren durchlaufen.

Insbesondere wenn das erfindungsgemäße Verfahren in Gegenwart eines unter Normalbedingungen gasförmigen Verdünnungsmittels durchgeführt und dieses rückgeführt werden soll, so ist es erforderlich, aus der Gasphase Kohlendioxid zu entfernen. Dies kann nach einem üblichen Absorptionsverfahren, z. B. durch eine Wäsche mit einem Amin, geschehen. Wird das erfindungsgemäße Verfahren mit

gasförmigen Kohlenwasserstoffen durchgeführt, so kann das entstandene Kohlendioxid in analoger Weise entfernt werden. Es ist jedoch auch möglich, das Kohlendioxid enthaltende Kohlenwasserstoffgas ohne Abtrennung des Kohlendioxids direkt als Heizmittel oder als Einsatz zur Reformierung zu verwenden. Wenn die Gasphase im wesentlichen nur Kohlendioxid enthält, so wird sie im allgemeinen verworfen, gegebenenfalls nach Abtrennung oder Zersetzung vorhandener organischer Bestandteile.

Eine andere Form der Aufarbeitung der Reaktionsprodukte ist die partielle Kondensation. Sie wird besonders vorteilhaft eingesetzt, wenn alle Reaktionsprodukte zunächst gasförmig vorliegen. Durch stufenweises Abkühlen und Kondensation können dann eine Gasphase und mehrere flüssige Phasen getrennt erhalten werden, die beispielsweise die als Verfahrensprodukt gewünschten Amine zusammen mit evtl. nicht umgesetzter Nitroverbindung, gegebenenfalls Kohlenwasserstoffe oder Kohlenwasserstoffgemische und Wasser in jeweils angereicherter Form enthalten.

Die erfindungsgemäß erhältlichen aromatischen Amine sind bekannte Zwischenprodukte, die z. B. in bekannter Weise zur Herstellung von Farbstoffen, Kunststoffen und Polyurethanen verwendet werden können (s. Ullmanns Encyclopädie der Technischen Chemie, Band 7, S. 401, Weinheim [1974]).

Das erfindungsgemäße Verfahren hat eine Reihe von Vorteilen. So kann der erfindungsgemäß einzusetzende Katalysator, im Vergleich zu den bisher verwendeten Katalysatoren, wesentlich länger betrieben werden, bis er regeneriert werden muß. Die Laufzeit des Katalysators zwischen zwei Regenerationen kann beispielsweise 200 und mehr Stunden betragen. Weiterhin haben die erfindungsgemäß einzusetzenden Katalysatoren eine hohe Gesamtlebenszeit, da sie nach der Regeneration im allgemeinen keine Aktivitätsverluste aufweisen. Schließlich gestattet das erfindungsgemäße Verfahren aromatische Amine in hohen Raum-Zeit-Ausbeuten und in höheren Selektivitäten als bisher herzustellen. Selbst zahlenmäßig geringe Selektivitätsverbesserungen sind bei den großen Mengen, in denen aromatische Amine hergestellt werden, von großer technischer und wirtschaftlicher Bedeutung. Außerdem kann das erfindungsgemäße Verfahren bei Temperaturen durchgeführt werden, bei denen die Gewinnung und Verwendung der Reaktionswärme besonders wirtschaftlich ist.

Es ist als ausgesprochen überraschend anzusehen, daß mit den erfindungsgemäßen Kupferchromit- bzw. Kupferchromit enthaltenden Katalysatoren diese Effekte auftreten, denn gemäß dem Stand der Technik werden mit Katalysatoren, die Kupfer und Chrom in anderer Form enthalten, diese Effekte nicht erzielt.

Das erfindungsgemäße Verfahren wird durch die nachstehenden Beispiele erläutert, ohne es darauf zu beschränken.

## Beispiele

In den Beispielen wurden folgende Katalysatoren eingesetzt:

### Katalysator A

Käuflicher Kupferchromit-Katalysator (Bayer-Katalysator 9127) in 5 × 5 mm Pellets, 46 Gew.-% Kupfer und 27 Gew.-% Chrom enthaltend.

### Katalysator B

300 g pulverförmiger Katalysator A wurden mit 300 ml Wasser versetzt, im Vakuum bei Raumtemperatur getrocknet und bei 300° C 8 Stunden lang mit einem Wasserstoffstrom von 30 l/h behandelt. Aus 80 Gewichtsteilen dieses Katalysatorpulvers wurden unter Verwendung von 20 Gewichtsteilen Pelletierungshilfsmitteln (Graphit, Magnesiumstearat und Aluminiumoxid) 3 × 5 mm Pellets gepreßt.

### Katalysator C

Dieser Katalysator wurde entsprechend dem Katalysator B hergestellt, jedoch unter Verwendung von Wasser, in dem 2,36 g Silbernitrat gelöst waren.

### Katalysator D

83,3 Gewichtsteile pulverförmiger Katalysator A und 16,7 Gewichtsteile Mangancarbonat wurden fein zerrieben, innig vermischt und 3 Wochen auf 300° C erwärmt. Von dem so erhaltenen Pulver wurden 90 Gewichtsteile zusammen mit 10 Gewichtsteilen Blauton (das ist eine sehr reine, praktisch schwermetallfreie Tonerde) unter Zugabe von etwas Wasser angeteigt und auf ein Lochblech ausgestrichen. Nach dem Trocknen an der Luft wurde 2 Stunden auf 150° C, anschließend 3 Stunden auf 500° C erhitzt. Danach wurden die 3 × 5 mm Pellets aus den Löchern gedrückt.

Katalysator E

76,4 Gewichtsteile Katalysator A, 9,9 Gewichtsteile alpha-$Fe_2O_3$ und 13,7 Gewichtsteile $Cu_2O$ wurden in fein pulverisierter Form 3 Wochen bei 800° C gesintert und danach mit 11,1 Gewichtsteilen Blauton, wie bei der Herstellung des Katalysators D angegeben, zu Pellets verarbeitet.

Katalysator F

Aus 88,5 Gewichtsteilen Katalysator A und 11,5 Gewichtsteilen NiO wurde nach der bei Katalysator E angegebenen Arbeitsweise ein Katalysator hergestellt und pelletiert.

Katalysator G

Käuflicher Kupferchromit-Katalysator, Alfa Products (Ventron) Katalog 1981, Nr. 11 842, 3× 4 mm Pellets. Nach Katalogangaben ist die Zusammensetzung dieses Katalysators umschrieben mit 33% CuO, 38% $Cr_2O_3$ und 9% BaO.

Katalysator H

Kupferchromit-Tränkkatalysator, hergestellt nach der oben angegebenen Literaturstelle von Laidig, Hönicke und Griesbaum auf 4 mm gamma-$Al_2O_3$-Kugeln.

Katalysator I

Käuflicher Kupferchromit-Katalysator, Alfa Products (Ventron) Katalog 1981, Nr. 11 845, 3 × 4 mm Pellets. Nach Katalogangaben ist die Zusammensetzung dieses Katalysators umschrieben mit 42%CuO und 38% $Cr_2O_3$.

Beispiel 1–11

Ein elektrisch beheizbarer Rohrreaktor aus Edelstahl von 25 mm Durchmesser wurde von unten nach oben mit 25 ml Porzellankugeln, 130 ml Katalysatorpellets und 45 ml Porzellankugeln gefüllt. Der Reaktoreingang wurde mit einem bei 300° C betriebenen Verdampfer verbunden. In diesen wurden über Dosierpumpen 41 g/h Nitrobenzol und 34 g/h Wasser eingespeist. Stickstoff und Methan wurden je mit 25 l/h über Rotameter dosiert und ebenfalls durch den Verdampfer in den Reaktor geleitet. Vom Reaktorausgang wurden die Reaktionsprodukte mit einem mit Kühlwasser betriebenen Kühler auf 50° C abgekühlt und in ein Abscheidegefäß geleitet, welches mit einem zweiten, mit einem Kühlmittel von −15° C betriebenen Kühler und einem Ablaßstutzen versehen war. Der Gasstrom wurde über den zweiten Kühler entnommen und über eine Gasuhr abgeleitet. Zur Gasprobenahme war ein Bypass vorhanden.

Zu Versuchbeginn wurde der Reaktor zunächst mit Stickstoff gespült und währenddessen der Reaktor und der Verdampfer aufgeheizt. Dann wurden nacheinander Methan, Wasser und Nitrobenzol zudosiert. Letzteres wurde innerhalb von einer Stunde auf die Solldurchsatzmenge gebracht und der dabei anfallende zweiphasige Produktvorlauf abgelassen und verworfen. Über einige Stunden wurde danach der Hauptlauf gesammelt, zur Homogenisierung mit der gleichen Gewichtsmenge Ethanol versetzt und gaschromatographisch quantitativ analysiert.

Die Reaktionstemperaturen wichen über die Katalysatorzone um etwa ±20° C von dem angegebenen Mittelwert ab. Weitere Einzelheiten sind in Tabelle 1 angegeben.

Tabelle 1

| Beispiel Nr. | Katalysator | Laufzeit (Std.) | Reaktionstemperatur (° C) | Umsatz (% Nitrobenzol) | Selektivität (Mol-% Anilin bezüglich Nitrobenzol) |
|---|---|---|---|---|---|
| 1 } *) | A | 4 | 300 | 41 | 98,9 |
| 2 } *) | A | 4,5 | 350 | 52 | 98,4 |

(Fortsetzung)

| Beispiel Nr. | Katalysator | Laufzeit (Std.) | Reaktionstemperatur (° C) | Umsatz (% Nitrobenzol) | Selektivität (Mol-% Anilin bezüglich Nitrobenzol) |
|---|---|---|---|---|---|
| 3 *) | B | 4 | 300 | 64 | 96,6 |
| 4 *) | B | 4 | 350 | 63 | 96,2 |
| 5 | C | 4,5 | 300 | 91 | 99,9 |
| 6 *) | D | 2,5 | 300 | 94 | 96,2 |
| 7 *) | D | 3,5 | 350 | 98 | 95,0 |
| 8 | E | 4 | 300 | 45 | 98,7 |
| 9 | F | 4,5 | 350 | 38 | 97,0 |
| 10 | G | 5,5 | 350 | 95 | 95,5 |
| 11 | H | 3 | 350 | 16 | 94,0 |

*) Diese Versuche wurden jeweils ohne Katalysatorwechsel und ohne Regeneration bei verschiedenen Temperaturen gefahren.

## Beispiel 12

In der gleichen Apparatur wie in den Beispielen 1—11 verwendet, wurde über den Katalysator I ein Durchsatz von 34 ml/h Wasser und 25 l/h Stickstoff eingestellt. Bei 350° C Reaktortemperatur wurden 88,3 g/h eines Gemisches nachstehender Zusammensetzung (Gew.-%) durchgesetzt: n-Pentan (10), n-Hexan (6), Cyclohexen (6), Cyclohexan (10), n-Heptan (18) und Nitrobenzol (50). Nach 5 Stunden Vorlauf wurden in einer 2,5 Stunden-Bilanz folgende Produkte erhalten: 77 g Wasserphase, 1,2 Gew.-% Anilin enthaltend, zwei organiche Phasen von zusammen 155 g, welche 6,1 g n-Pentan, 9,7 g n-Hexan, 4,5 g Benzol, 11,5 g Cyclohexen, 10,9 g Cyclohexan, 27,1 g n-Heptan, 74,1 g Anilin und 7,4 g Nitrobenzol (gemäß gaschromatographischer Analyse) enthielten. Bei Raumtemperatur gemessen fielen in der Bilanzzeit 82 l eines Produktgases an. Eine Gasprobe, welche vor Beendigung des Versuches genommen wurde, wies nach gaschromatographischer Analyse folgende Zusammensetzung auf: 64,5 Vol.-% Stickstoff, 0,3 Vol.-% Kohlenmonoxid, 18,7 Vol.-% Kohlendioxid, 2,8 Vol.-% Kohlenwasserstoffe $C_1$—$C_4$ und 12,8 Vol.-% Kohlenwasserstoffe $C_5$—$C_7$. Der Umsatz an Nitrobenzol betrug 93%, die Selektivität der Anilinbildung bezogen auf Nitrobenzol 95%.

## Beispiel 13

In entsprechender Arbeitsweise wie bei den Beispielen 1 bis 11, jedoch mit 25 l/h Propan anstatt Methan, wurde 5 Stunden lang Nitrobenzol an Katalysator I bei 400° C umgesetzt. Der Nitrobenzolumsatz betrug 74%, die Anilinselektivität 95%. Eine gegen Ende des Versuchs genommene Gasprobe wies folgende Zusammensetzung (Vol.-%) auf: $C_2$ (0,9), Propan (44,3), Propylen (0,3), $C_4$ (1,6), CO (0,02), $CO_2$ (8,2), $H_2$ (0,003), Methan (0,3) $N_2$ (43,9).

## Beispiel 14

Ein Rohrreaktor aus Edelstahl mit 19 mm Innendurchmesser, versehen mit 3 elektrischen Heizkreisen und 5 Temperaturmeßstellen wurde in eine Versuchsapparatur eingebaut, die der für die Beispiele 1 bis 11 verwendeten entsprach. Die Temperaturmeßstellen waren gleichmäßig über eine Schüttung verteilt, die im unteren Teil aus 130 ml des Katalysators I und im oberen Teil aus 40 ml Porzellansattelkörpern bestand. Bei der Versuchsausführung wurden die Reaktionstemperaturen in einem Intervall von 15° C gehalten. Eingesetzt wurden Methan und Stickstoff, wie in Tabelle 2 angegeben, sowie 41 g/h Nitrobenzol und 34 g/h Wasser.

Es wurde dann über einen Zeitraum von 1200 Stunden so verfahren, wie in der Tabelle 2 angegeben und die ebenfalls in Tabelle 2 angegebenen Umsätze an Nitrobenzol erhalten.

Tabelle 2

| Zeit | Reaktionstemperatur | Umsatz an Nitrobenzol (%) | | Stickstoffmenge | Methanmenge | Bemerkungen |
|---|---|---|---|---|---|---|
| (Stunden) | (° C) | Anfang | Ende | (l/h) | (l/h) | |
| 0–510 | 390–420 | | | 25 | 25 | zwischenzeitlich 5 Regenerationen*) |
| 510–524 | | | | | | Regeneration*) |
| 524–740 | 400 | 77 | 67 | 25 | 25 | |
| 740–754 | | | | | | Regeneration*) |
| 754–938 | 400 | 90 | 75 | 25 | 25 | |
| 938–952 | | | | | | Regeneration*) |
| 952–996 | 400 | 90 | | 25 | 25 | keine Regeneration |
| 996–1200 | 400 | | 72 | 37,5 | 12,5 | desgl. |

*) Details s. weiter unten.

Das Abklingen der Katalysatoraktivität (= Umsatzrückgang des Nitrobenzols) in den Zeiträumen 524–740, 754–938 und 952–1200 Stunden verlief immer nahezu linear, auch bei der Zurücknahme der Methaneinspeisung zugunsten einer erhöhten Stickstoffeinspeisung nach 996 Stunden.

In den drei ab 524 Stunden beschriebenen Produktionsphasen lag die Selektivität der Anilinbildung konstant bei 96% (bezogen auf umgesetztes Nitrobenzol) bzw. bei 98 bis 99% (bezogen auf umgesetztes Methan). Bezogen auf umgesetztes Nitrobenzol wurde 2 bis 3% Azobenzol erhalten.

Durch erneute Regeneration nach 1200 Betriebsstunden wurde der Katalysator wieder reaktiviert und erreichte wieder einen Nitrobenzolumsatz von 90%.

## Regeneration

Bei Reaktionstemperatur wurde die Zufuhr von Nitrobenzol und Methan abgestellt. Der Stickstoffeinsatz wurde auf 50 l/h verdoppelt. Nach einer Stunde wurde begonnen, Luft zuzuspeisen und entsprechend wurde der Stickstoffeinsatz zurückgenommen bis nach 5 Stunden der Gaseinsatz auf 25 l/h Luft und 25 l/h Stickstoff eingestellt war. 8 Stunden später wurde der Luftstrom durch einen zusätzlichen Stickstoffstrom ersetzt, danach wurde die Zufuhr von Nitrobenzol und Methan wieder geöffnet und der ursprüngliche Gasdurchsatz wieder eingestellt. Der Nitrobenzoleinsatz wurde in 5 Stunden wieder auf den Solleinsatz gebracht.

## Beispiel 15

Entsprechend Beispiel 1 wurde eine vergrößerte Versuchsapparatur aufgebaut, deren Rohrreaktor bei 25 mm Innendurchmesser 3 m lang war und mit 8 Temperaturfühlern sowie einem Druckregelventil am Reaktorausgang ausgestattet war. Die Beheizung erfolgte indirekt über eine umlaufende Salzschnecke. Der Reaktor wurde mit einer Schüttung von 1100 ml Katalysator I und darüber mit 350 ml Porzellansattelkörpern beschickt. Bei 3 bar Reaktordruck und 400 ± 10° C Reaktortemperatur sowie 900 l/h Methandurchsatz wurde ein Einsatz von 550 g/h Wasser und 550 g/h Nitrobenzol eingestellt. Der Nitrobenzolumsatz betrug anfangs 98% und fiel innerhalb von 180 Std. etwa linear auf 78% ab. Die Selektivität der Anilinbildung bezogen auf Nitrobenzol lag bei 95 ± 1 Mol-%. Als Nebenprodukte wurden jeweils 2–3 Mol-% Benzol und Azobenzol erhalten. Nach einer Regeneration, die wie in Beispiel 14 beschrieben mit – entsprechend der Reaktorgröße – vergrößertem Gasdurchsatz durchgeführt worden war, stellten sich wieder die ursprünglichen Werte für Umsatz und Selektivität ein.

**Patentansprüche**

1. Verfahren zur Herstellung von primären aromatischen Aminen durch Umsetzung von aromatischen Nitroverbindungen mit Kohlenwasserstoffen, dadurch gekennzeichnet, daß man Kohlenwasserstoffe mit einem Molekulargewicht bis zu 170 und einer Zusammensetzung $C_mH_n$ mit Werten von $\frac{m}{n}$ von 0,25 bis 0,60 oder Kohlenwasserstoffgemische mit einem entsprechenden mittleren Molekulargewicht und einer entsprechenden mittleren Zusammensetzung in Gegenwart von Wasser oder Wasserdampf einsetzt und die Umsetzung in Gegenwart eines Katalysators durchführt, welcher Kupfer, Chrom und daran chemisch gebundenen Sauerstoff ganz oder teilweise in Form von Kupferchromiten enthält.

2. Verfahren nach Anspruch 1, dadurch gekennzeichnet, daß man die Umsetzung in Gegenwart von Katalysatoren durchführt, deren Gesamtzusammensetzung der summarischen Formel

$$xCuO \cdot (1-x)Cr_2O_3$$

entspricht, wobei x Gewichtsanteile bedeutet und für Werte von 0,2 bis 0,9 steht.

3. Verfahren nach Ansprüchen 1 und 2, dadurch gekennzeichnet, daß der Katalysator als weitere Komponenten ein Element mit den Ordnungszahlen 25 bis 28, 30 oder 56 in oxidischer Form und/oder ein Element mit den Ordnungszahlen 44 bis 47 oder 75 bis 79 in metallischer Form enthält.

4. Verfahren nach Ansprüchen 1 bis 3, dadurch gekennzeichnet, daß der Katalysator mit inerten Trägern, Zusatzstoffen, organischen Bindemitteln und/oder Porosierungsmitteln verarbeitet wurde.

5. Verfahren nach Ansprüchen 1 bis 4, dadurch gekennzeichnet, daß man als Kohlenwasserstoff Methan, natürliches Erdgas und/oder SNG (substitute natural gas) und als aromatische Nitroverbindung Nitrobenzol oder 1,3-Dinitrobenzol einsetzt.

6. Verfahren nach Ansprüchen 1 bis 5, dadurch gekennzeichnet, daß man Wasser oder Wasserdampf in der 0,1- bis 3fachen Gewichtsmenge, bezogen auf die eingesetzte aromatische Nitroverbindung, zuführt und gegebenenfalls ein inertes Verdünnungsmittel mit verwendet.

7. Verfahren nach Ansprüchen 1 bis 6, dadurch gekennzeichnet, daß man es in der Gasphase und bei Reaktionstemperaturen im Bereich 250 bis 450° C durchführt und Drucke im Bereich Normaldruck bis zu 50 bar anwendet.

8. Verfahren nach Ansprüchen 1 bis 7, dadurch gekennzeichnet, daß man den Katalysator in Gegenwart von Wasserdampf regeneriert, wenn er im Laufe der Zeit in der Aktivität und/oder Selektivität abfällt, wobei man molekularen Sauerstoff enthaltende Gase unter Zusatz von Wasserdampf bei erhöhter Temperatur über den Katalysator leitet.

9. Verfahren nach Ansprüchen 1 bis 8, dadurch gekennzeichnet, daß man nach der Reaktion die Reaktionsgemische abkühlt und die dabei anfallende Phasen trennt und weiter aufarbeitet.

10. Verfahren nach Anspruch 9, dadurch gekennzeichnet, daß man aus der anfallenden Gasphase Kohlendioxid entfernt und das Restgas zurückführt oder die anfallende Gasphase ohne Abtrennung des Kohlendioxids direkt als Heizmittel oder als Einsatz zur Reformierung verwendet.

**Claims**

1. Process for preparing primary aromatic amines by reacting aromatic nitro compounds with hydrocarbons, characterised in that hydrocarbons having a molecular weight of up to 170 and a $C_mH_n$ composition with values $\frac{m}{n}$ of 0.25 to 0.60 or hydrocarbon mixtures having a corresponding mean molecular weight and a corresponding mean composition are used in the presence of water or steam and the reaction is carried out in the presence of a catalyst which contains copper, chromium and oxygen chemically bended thereto wholly or partially in the form of copper chromites.

2. Process according to Claim 1, characterised in that the reaction is carried out in the presence of catalysts whose overall composition corresponds to the empirical formula

$$xCuO \cdot (1-x)Cr_2O_3$$

where x denotes proportions by weight and represents values of 0.2 to 0.9.

3. Process according to Claims 1 and 2, characterised in that the catalyst contains as further components an element having an atomic number of 25 to 28, 30 or 56 in oxidic form and/or an element having an atomic number of 44 to 47 or 75 to 79 in metallic form.

3. Process according to Claims 1 and 2, characterised in that the catalyst contains as further components an element having an atomic number of 25 to 28, 30 or 56 in oxidic form and/or an element having an atomic number of 44 to 47 or 75 to 79 in metallic form.

4. Process according to Claims 1 to 3, characterised in that the catalyst was processed together with inert supports, additives, organic binders and/or porosity-inducing agents.

5. Process according to Claims 1 to 4, characterised in that methane, natural gas and/or SNG (substitute natural gas) are used as the hydrocarbon and nitrobenzene or 1,3-dinitrobenzene is used as the aromatic nitro compound.

6. Process according to Claims 1 to 5, characterised in that water or steam is supplied in a weight 0.1 to 3 times that of the aromatic nitro compound used, and an inert diluent is concomitantly used if desired.

7. Process according to Claims 1 to 6, characterised in that the process is carried out in the gas phase and at reaction temperatures within a range of 250 to 450° C and under pressures within a range of normal pressure up to 50 bar.

8. Process according to Claims 1 to 7, characterised in that the catalyst is regenerated in the presence of steam when its activity and/or selectivity decreases in the course of time, and the regeneration is carried out by passing gases containing molecular oxygen and added steam over the catalyst at an elevated temperature.

9. Process according to Claims 1 to 8, characterised in that, after the reaction, the reaction mixtures are cooled down and the resulting phases are separated from one another and further worked up.

10. Process according to Claim 9, characterised in that carbon dioxide is removed from the gas phase obtained and the residual gas is recycled, or the gas phase obtained is used directly, without the carbon dioxide being separated off, as a heating medium or as a feed in reforming.

**Revendications**

1. Procédé de production d'amines aromatiques primaires par réaction de composés nitrés aromatiques avec des hydrocarbures, caractérisé en ce qu'on utilise des hydrocarbures de poids moléculaire allant jusqu'à 170 et de composition $C_mH_n$ avec des valeurs de $\frac{m}{n}$ de 0,25 à 0,60 ou des mélanges d'hydrocarbures de poids moléculaire moyen correspondant et de composition moyenne correspondante en présence d'eau ou de vapeur d'eau, et on conduit la réaction en présence d'un catalyseur qui contient du cuivre, du chrome et de l'oxygène lié chimiquement à ces métaux, en totalité ou en partie sous la forme de chromites de cuivre.

2. Procédé suivant la revendication 1, caractérisé en ce qu'on conduit la réaction en présence de catalyseurs dont la composition totale répond à la formule brute

$$xCuO \cdot (1-x)Cr_2O_3$$

dans laquelle x désigne des proportions en poids et a des valeurs de 0,2 à 0,9.

3. Procédé suivant les revendications 1 et 2, caractérisé en ce que le catalyseur contient comme autres composants un élément ayant les nombres atomiques de 25 à 28, 30 ou 56 sous la forme oxydique et/ou un élément ayant les nombres atomiques de 44 à 47 ou 75 à 79 sous la forme métallique.

4. Procédé suivant les revendications 1 à 3, caractérisé en ce que le catalyseur a été traité avec des supports inertes, des additifs, des liants organiques et/ou des agents de porosité.

5. Procédé suivant les revendications 1 à 4, caractérisé en ce qu'on utilise comme hydrocarbure le méthane, le gaz naturel et/ou le SNG (substitute natural gas) et comme composé nitré aromatique, le nitrobenzène ou le 1,3-dinitrobenzène.

6. Procédé suivant les revendications 1 à 5, caractérisé en ce qu'on fait arriver l'eau ou la vapeur d'eau en quantité en poids de 0,1 à 3 fois la quantité utilisée de composé nitré aromatique et on utilise, le cas échéant, en même temps un diluant inerte.

7. Procédé suivant les revendications 1 à 6, caractérisé en ce qu'on le met en oeuvre en phase gazeuse et à des températures de réaction comprises dans l'intervalle de 250 à 450° C et on utilise des pressions dans l'intervalle allant de la pression normale à 50 bars.

8. Procédé suivant les revendications 1 à 7, caractérisé en ce qu'on régénère le catalyseur en présence de vapeur d'eau lorsque son activité et/ou sa sélectivité décroissent ou cours du temps, en faisant passer sur le catalyseur des gaz contenant de l'oxygène moléculaire avec addition de vapeur d'eau, à température élevée.

9. Procédé suivant les revendications 1 à 8, caractérisé en ce qu'on refroidit les mélanges réactionnels après la réaction et en ce qu'on sépare et traite ultérieurement les phases qui sont alors obtenues.

10. Procédé suivant la revendication 9, caractérisé en ce qu'on élimine l'anhydride carbonique de la phase gazeuse obtenue et on recycle le gaz résiduel, ou bien on utilise la phase gazeuse obtenue, sans séparation de l'anhydride carbonique, directement comme fluide de chauffage ou comme charge de reformage.